# EUROPEAN PATENT APPLICATION

(11) **EP 4 570 912 A1**
(43) Date of publication of application: **18.06.2025**
(21) Application number: 23851911.0
(22) Date of filing: 10.08.2023
(51) Int. Cl.: C12N 15/63, C12N 15/10, A61K 48/00, C12P 19/34

(54) **METHOD FOR PREPARING LINEAR CLOSED DNA AND PLASMID FOR USE IN THE METHOD**

(30) Priority: 10.08.2022 CN 202210955391
(71) Applicant: Zhenjiang Probio Biotech Co., Ltd., Zhenjiang, Jiangsu 212009 (CN)
(72) Inventor: CHEN, Junpeng, Zhenjiang, Jiangsu 212009 (CN); ZHANG, Xinmiao, Zhenjiang, Jiangsu 212009 (CN)
(74) Representative: Brann AB
(86) International application number: PCT/CN2023/112131
(87) International publication number: WO 2024/032690

(57) **Abstract**

Provided are a method for preparing a linear closed DNA and a plasmid for use in the method. The plasmid comprises two editable regions of prokaryotic telomerase target sequences which are connected in tandem in the same direction, and both ends of the editing regions are independently provided with one or more restriction endonuclease digestion sites. The method can yield high-purity LcDNA and can be better applied to clinical research and commercial applications.

## Description

### Cross Reference to Related Applications

The present application claims the priority of Chinese patent application no. 202210955391.7 filed on August 10, 2022, which is incorporated herein by reference in its entirety.

### Technical Field

The present invention relates to the field of genetic engineering technologies, in particular to a method for preparing a linear closed DNA and a plasmid for use in the method.

### Background Art

Gene therapy is a method for preventing or treating a disease by repairing, replacing, or regulating a gene using a nucleic acid drug. Delivery vectors for gene therapy can be generally divided into viral vectors and non-viral vectors. The viral vectors are widely used in the fields of cell therapy and gene therapy, due to their natural ability to infect cells and deliver genes efficiently. However, the use of viral vectors also has risks to varying degrees. Lentiviral and retroviral vectors are mainly used to integrate a gene of interest into a cell genome, which also causes high-risk gene mutations in the cell. The risk of insertion mutations caused by adenoassociated viral vectors is low, but it still exists. Adenoviral vectors can exist episomally, independent of the genome; however, they may cause toxicity and immunogenicity in cells. Many people have pre-existing immunity to some viral vectors such as AdV5 or AAV2 due to their wide distribution, so such viral vectors cannot be used as gene delivery vectors. Despite low seropositivity rates in humans, all these viral vector families cannot be used repeatedly as gene delivery vectors due to the development of immunity to them in humans. However, non-viral vectors have much lower cytotoxicity and immunogenicity than viral vectors, so non-viral vectors are also gaining increasing attention in the clinical research and commercial use of gene therapy and cell therapy.

Conventional non-viral vectors are mainly plasmids. A plasmid vector is mainly composed of a prokaryotic replicon, a resistance gene, a gene of interest, and other elements. However, the prokaryotic replicon, resistance gene, and CpG sequence on the plasmid vector may cause unnecessary immune response in cells and cell canceration. Therefore, scientists invented a method for producing a linear closed DNA (LcDNA) by using prokaryotic telomerase TelN derived from *Escherichia coli* phage N15. The LcDNA only carries the gene of interest and TelN recognition sites at both ends, and sequences that do not need to be delivered into cells such as the prokaryotic replicon and the resistance gene are effectively removed from the plasmid. Compared with conventional plasmid vectors, LcDNAs show a comparable or even better expression level *in vitro* or *in vivo* and are safer and more efficient in transfection efficiency.

Small-scale production of LcDNAs is not difficult; however, large-scale industrial production under GMP conditions encounters numerous challenges, such as the oversized and non-universal circular plasmid backbones for producing LcDNAs; instability in large-scale digestion; insufficient purity to meet clinical grade, and potential introduction of organic reagents such as Triton, isopropanol, and ethanol, with a high residual risk; and a lower fidelity of polymerase Phi29 during *in vitro* amplification compared to *in vivo* replication in *E. coli,* which may result in unnecessary mutations, etc.

### Summary of the Invention

The present invention relates to a plasmid, wherein the plasmid comprises an editable region of two prokaryotic telomerase target sequences which are connected in tandem in the same direction, and both ends of the editing regions are independently provided with one or more restriction endonuclease digestion sites.

In some embodiments, the length of the above plasmid is ≤ 2500 bp, or ≤ 2300 bp, or ≤ 2100 bp. In some preferred embodiments, the length of the above plasmid is ≤ 2100 bp.

In some embodiments, the above plasmid further comprises a prokaryotic replicon and a selectable gene.

In some embodiments, the restriction endonuclease digestion site at each end is selected from any one or more of: digestion sites of AccI, AflII, AgeI, ApaI, AscI, AvrII, BamHI, BglI, BglII, BsaI, BspQI, BstBI, BsteII, ClaI, EcoNI, EcoRI, EcoRV, FseI, HindIII, KpnI, MfeI, MluI, NcoI, NdeI, NheI, NotI, PacI, PstI, PvuI, PvuII, SacI, SacII, SalI, ScaI, SmaI, SpeI, StuI, SwaI, XbaI, XhoI, and XmaI, preferably from any one or more of digestion sites of HindIII, SalI, SmaI, BamHI, EcoRV, XbaI, KpnI, and EcoRI. In some preferred embodiments, the digestion sites of HindIII, SalI, SmaI, and BamHI are inserted near one end of the editable region, and the digestion sites of EcoRV, XbaI, KpnI, and EcoRI are inserted near the other end of the editable region.

In some embodiments, the prokaryotic telomerase target sequence comprises a sequence set forth in SEQ ID NO: 1 or a nucleotide sequence that has at least 80%, at least 85%, at least 90%, at least 93%, at least 95%, at least 97%, or at least 99% identity with the sequence set forth in SEQ ID NO: 1 and is capable of functioning as a telomerase. In some preferred embodiments, the prokaryotic telomerase target sequence is as set forth in SEQ ID NO: 1.

In some embodiments, the above plasmid comprises a nucleotide sequence that has at least 80%, at least 85%, at least 90%, at least 93%, at least 95%, at least 97%, or at least 99% identity with the sequence set forth in SEQ ID NO: 2. In some preferred embodiments, the sequence of the above plasmid is as set forth in SEQ ID NO: 2.

The present invention further relates to a recombinant plasmid, wherein a gene of interest is inserted between the two prokaryotic telomerase target sequences of the plasmid as described above.

The present invention further relates to a method for preparing a linear closed DNA comprising:
a) digesting a recombinant plasmid comprising a gene of interest with a prokaryotic telomerase to separate the gene of interest from a heterogeneous nucleic acid in the recombinant plasmid;
b) using at least two restriction endonucleases for digestion such that the ends of the heterogeneous nucleic acid are exposed, and removing the heterogeneous nucleic acid by using an exonuclease; and
c) separating and purifying the linear closed DNA containing the gene of interest;
wherein the recombinant plasmid comprises prokaryotic telomerase target sequences and restriction endonuclease digestion sites, and the gene of interest is inserted between the prokaryotic telomerase target sequences.

In some embodiments, the recombinant plasmid mentioned in the above preparation method may be a plasmid in which a gene of interest is inserted between the two prokaryotic telomerase target sequences of the plasmid as described above.

In some embodiments, the exonuclease in the above method includes Exonuclease III and/or T5 Exonuclease. In some embodiments, the added amount of the exonuclease is 3000-6000 U/mg. In some specific embodiments, for the exonuclease, the digestion temperature is 34-40°C, and the digestion time is 2-12 hours; preferably, the digestion time is 3-6 hours. In some other specific embodiments, after the digestion with the exonuclease, EDTA with a final concentration of 1-50 mM is added and the temperature is raised to 70-80°C for 5-30 minutes of treatment. In some specific embodiments, after finishing the digestion with the prokaryotic telomerase and/or the digestion with the restriction endonuclease, the temperature is raised to 70-80°C for 5-30 minutes of treatment.

In some embodiments, the separation and purification method in the above preparation method comprises one or any combination of gel filtration chromatography, anion exchange chromatography, concentration and liquid exchange, and sterilization filtration. In some preferred embodiments, the separation and purification method comprises sequentially gel filtration chromatography and anion exchange chromatography.

In some specific embodiments, a packing for the gel filtration chromatography is Bestarose 6FF, and an eluate from the first ultraviolet peak with an absorbance of ≥ 50 mAu is collected.

In some other specific embodiments, a packing for the anion exchange chromatography is Fractogel EMD DEAE. In some preferred embodiments, loading conditions for the anion exchange chromatography comprise: using a loading solution containing 30%-70% chromatographic solution B, with a loading capacity of 0.2-1.0 mg/mL and a loading flow rate of ≤ 120 cm/h, wherein the chromatographic solution B comprises 0.5-1.5 M NaCl, 5-15 mM EDTA, and a buffer substance, pH 7.2-7.8. In some preferred embodiments, elution conditions for the anion exchange chromatography comprise: elution with 65%-100% chromatographic solution B at an elution flow rate of 30-120 cm/h, and collection of an eluate with an absorbance of ≥ 100 mAu.

The present invention further relates to a kit comprising a prokaryotic telomerase and the plasmid as described above;
or comprising a prokaryotic telomerase and the recombinant plasmid as described above.

In some embodiments, the kit further comprises a restriction endonuclease and/or an exonuclease, wherein the restriction endonuclease is capable of recognizing the restriction endonuclease digestion site.

The present invention further provides a linear closed DNA prepared by the above preparation method.

In the present invention, the plasmid produced by bacterial fermentation is used as a starting raw material, which is not obtained by amplification methods involving polymerases such as Phi29 and thus has a higher fidelity; in addition, by means of a specific design, the plasmid can maintain a sufficiently small size, thereby improving the total yield of LcDNA production from the source.

### Brief Description of the Drawings

In order to illustrate the technical solutions in the Detailed Description of Embodiments of the present invention or in the prior art more clearly, the accompanying drawings required in the description of the Detailed Description of Embodiments or the prior art are briefly described below. It is clear that the accompanying drawings in the following description illustrate some embodiments of the present invention, and those of ordinary skill in the art may further derive other drawings from these accompanying drawings without involving any inventive effort.
FIG. 1 is a schematic flow diagram of a process for producing and purifying LcDNAs;
FIG. 2A is a map of universal vector backbone, CFL Universal;
FIG. 2B is a complete sequence map of the vector of FIG. 2A in which a sequence of interest (GOI) has been inserted; wherein Kan promoter represents kanamycin gene promoter, KanR represents kanamycin resistance gene, pUC origin represents pUC promoter, MCS represents a multiple cloning site; GOI represents a sequence of interest; and telR and telL represent a prokaryotic telomerase target sequence;
FIG. 3 shows the detection of LcDNAs by agarose gel electrophoresis (AGE), wherein panels A and B are AGE detection patterns of intermediate samples and finished products of LcDNAs upon digestion and purification of a batch of 60 mg of circular plasmids containing a sequence of interest, and panel C is an AGE detection pattern of intermediate samples and finished products of LcDNAs upon digestion and purification of another batch of 60 mg of circular plasmids containing a sequence of interest, wherein
   in panels A-C, lane M1 represents superhelix DNA Ladder; lane M2 represents 1 kb DNA Ladder;
   Panel A: lane 1 represents the TelN digestion product; lane 2 represents the exonuclease enzyme digestion product; lane 3 represents the GF chromatography product; and lane 4 represents the AEX chromatography product;
   Panel B: lane 1 represents the finished product of LcDNAs; lane 2 represents 1xTAE buffer;
   Panel C: lane 1 represents the TelN digestion product; lane 2 represents the exonuclease enzyme digestion product; lane 3 represents the GF chromatography product; and lane 4 represents the AEX chromatography product; lane 5 represents the finished product of LcDNAs;

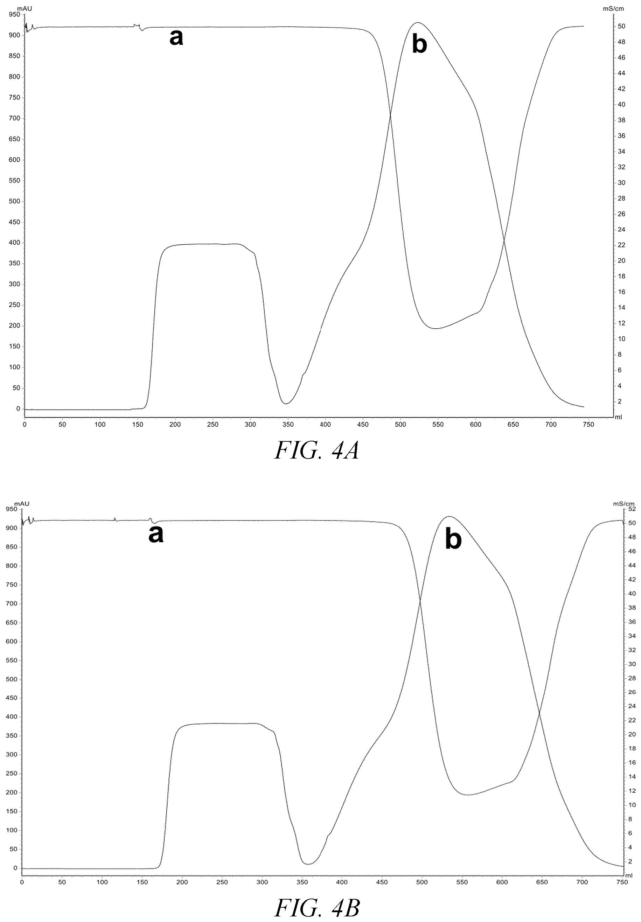

FIGs. 4A to 4E are gel filtration chromatograms of LcDNAs; wherein FIGs. 4A and 4B are gel filtration chromatograms of LcDNAs after digestion of two batches of 60 mg of circular plasmids containing the sequence of interest respectively, FIG. 4C is a gel filtration chromatogram of LcDNAs after digestion of a batch of 100 mg of circular plasmids containing the sequence of interest, and FIGs. 4D and 4E are gel filtration chromatograms of LcDNAs after digestion of two batches of 150 mg of circular plasmids containing the sequence of interest;

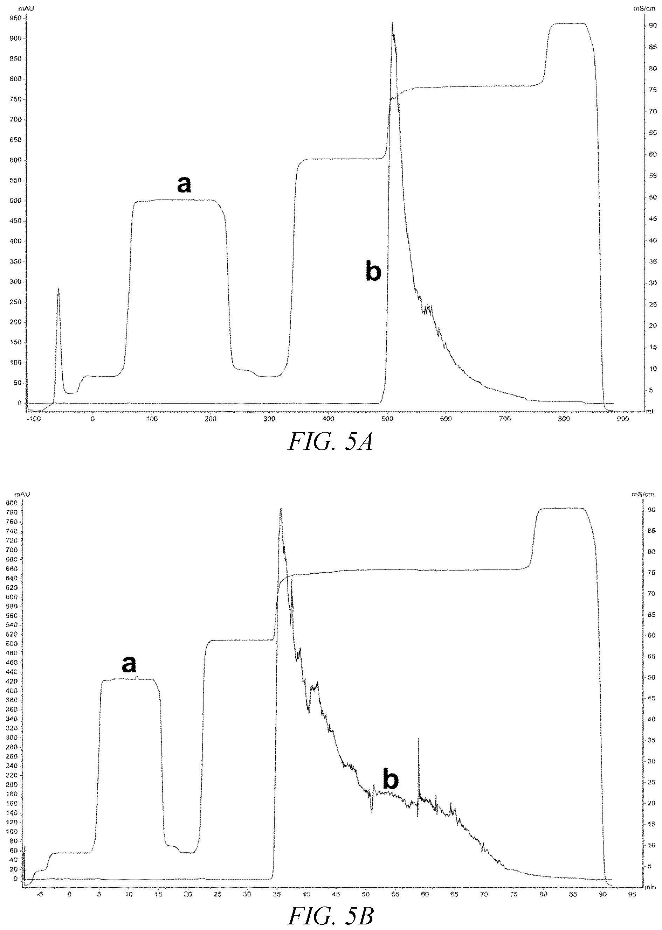

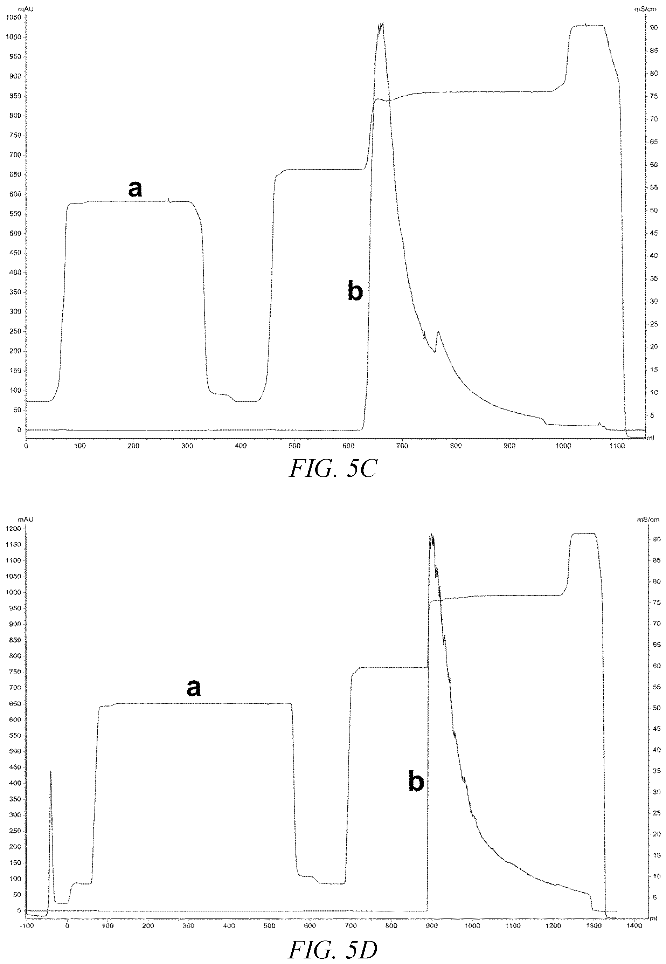

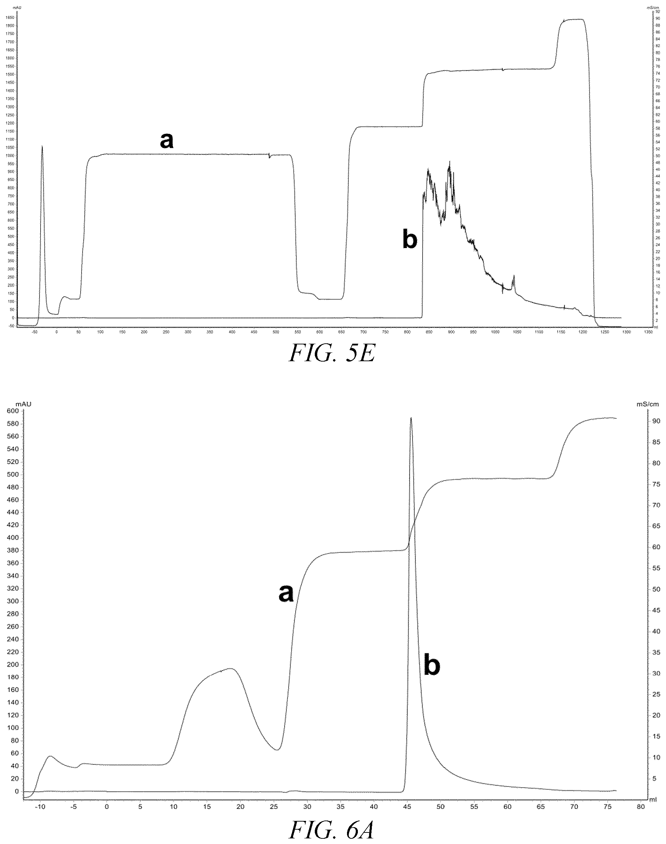

FIGs. 5A to 5E are anion exchange chromatograms of LcDNAs, wherein FIGs. 5A and 5B are anion exchange chromatograms of LcDNAs from two batches of 60 mg of circular plasmids containing the sequence of interest respectively, FIG. 5C is an anion exchange chromatogram of LcDNAs from a batch of 100 mg of circular plasmids containing the sequence of interest, and FIGs. 5D and 5E are anion exchange chromatograms of LcDNAs from two batches of 150 mg of circular plasmids containing the sequence of interest respectively;

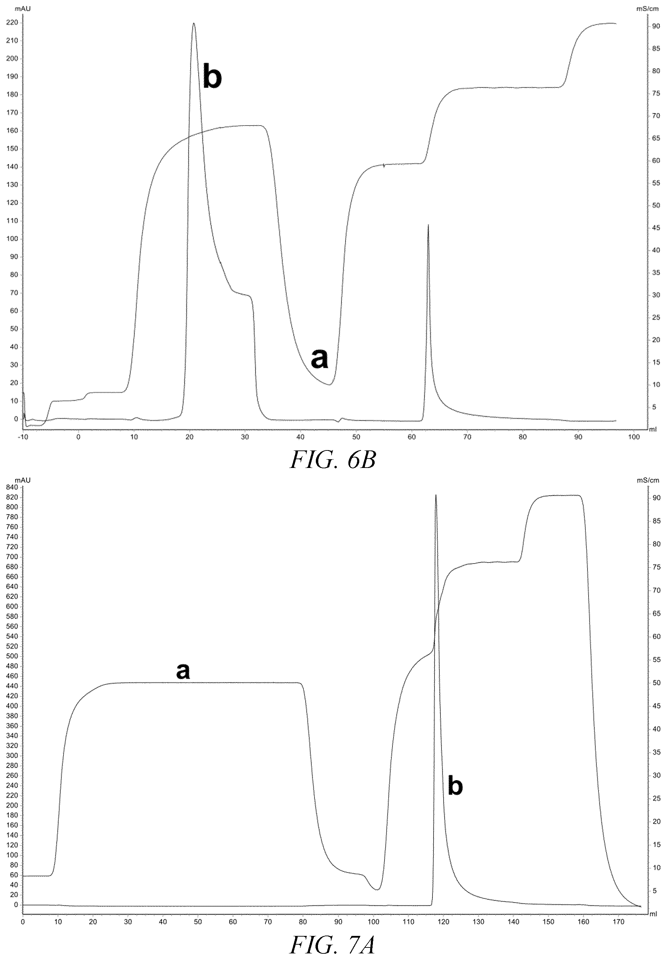

FIGs. 6A and 6B are anion exchange chromatograms of LcDNAs during the optimization of loading conditions, wherein FIG. 6A involves a loading solution containing 30% chromatographic solution B, and FIG. 6B involve a loading solution containing 70% chromatographic solution B;

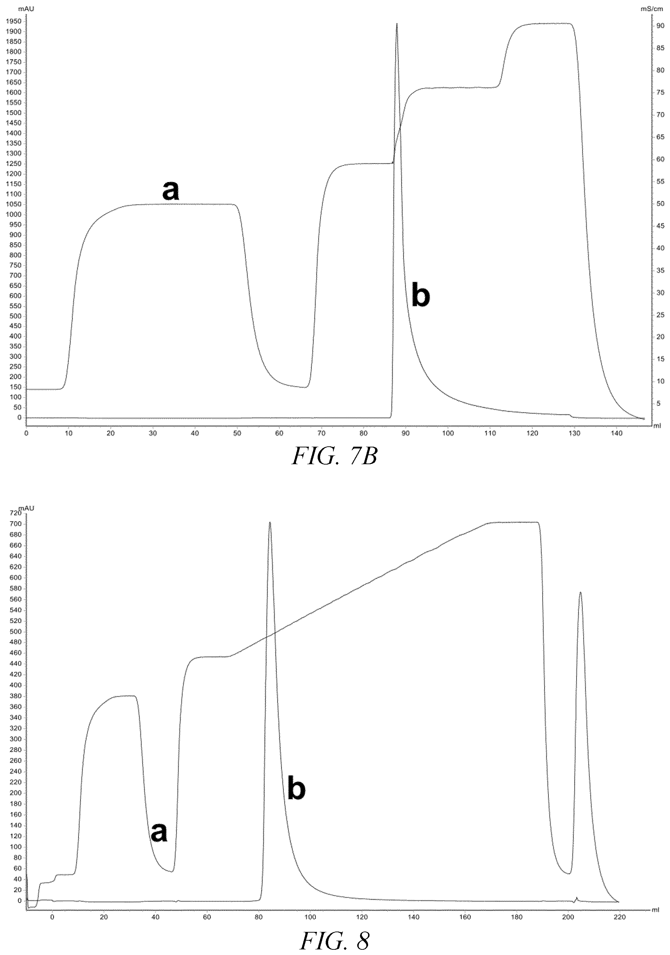

FIGs. 7A and 7B are anion exchange chromatograms of LcDNAs during the optimization of loading capacities, wherein the loading capacity in FIG. 7A is 0.25 mg/mL, and the loading capacity in FIG. 7B is 1.0 mg/mL; and
FIG. 8 shows anion exchange chromatography of LcDNAs with linear elution (gradient elution with 0-100% chromatographic solution B), wherein the maximum ultraviolet peak appears in the chromatogram upon elution with 67.5% chromatographic solution B.

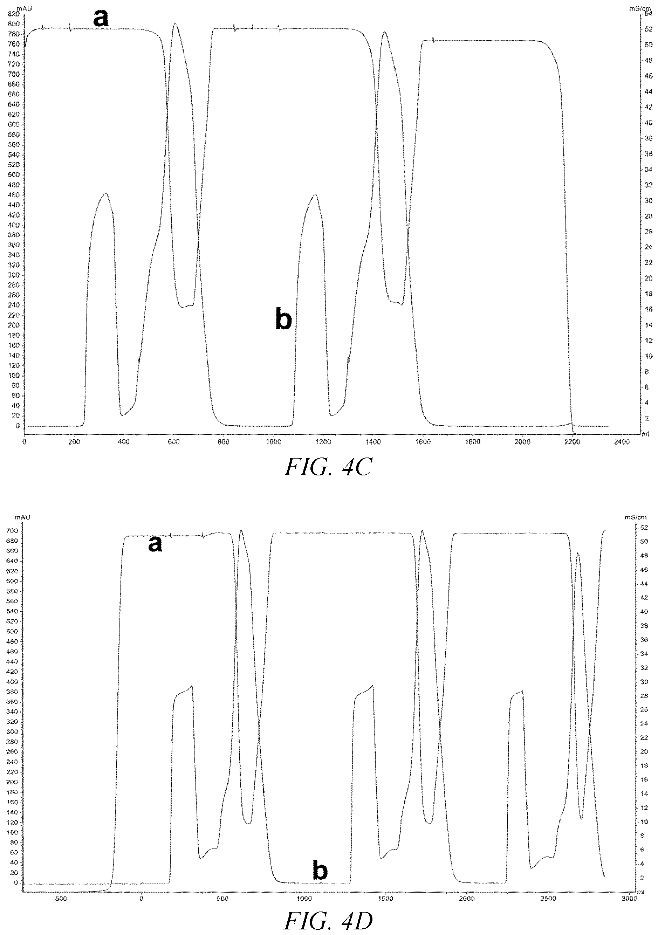

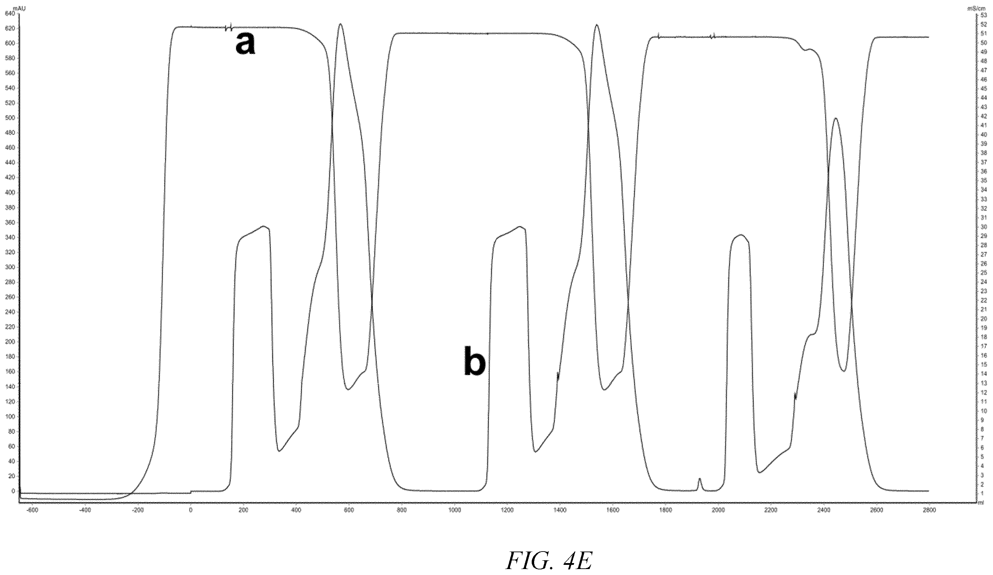

In FIGs. 4 to 8, all the curves marked with "a" represent electric conductivity and all the curves marked with "b" represent ultraviolet peaks.

### Detailed Description of Embodiments

Reference now will be made in detail to the embodiments of the present invention, one or more examples of which are set forth below. Each example is provided as an explanation rather than limiting the present invention. Indeed, it would have been obvious to a person skilled in the art that various modifications and variations may be made to the present invention without departing from the scope or spirit of the present invention. For instance, features illustrated or described as part of one embodiment, can be used on another embodiment to yield a still further embodiment.

Unless otherwise stated, all terms (including technical and scientific terms) used to disclose the present invention have the same meaning as commonly understood by those of ordinary skill in the art to which the present invention belongs. With further guidance, ensuing definitions are used to better understand the teachings of the present invention. Herein, the terms used in the description of the present invention are merely for the purpose of describing specific examples, but are not intended to limit the present invention.

The terms "and/or" and "or/and" used herein are selected to encompass any one of two or more associated items listed therein, as well as any and all combinations of the associated items listed therein, wherein the combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

The terms "contain", "comprise", and "include" used herein are synonymous, inclusive or open-ended, and do not exclude additional, unrecited members, elements, or method steps.

Numerical ranges expressed by endpoints in the present invention include all numbers and fractions included within the range, as well as the recited endpoints.

The present invention relates to a concentration value, and the fluctuations of the value are within a certain range. For example, it can fluctuate within the corresponding precision range. For example, with regard to the value 2%, fluctuations within the range of ± 0.1% may be permitted. For larger values or values that do not require way too fine control, the meaning is also allowed to include larger fluctuations. For example, with regard to the value 100 mM, fluctuations within the range of ± 1%, ± 2%, ± 5%, etc. may be permitted. When a molecular weight is involved, the meaning of its value is allowed to include fluctuations with the range of ± 10%.

In the present invention, expressions involving terms such as "a plurality of" and "multiple" refer to a quantity greater than or equal to 2, unless otherwise specified.

In the present invention, the technical features described in an open-ended manner include both a closed technical solution consisting of the listed features and an open-ended technical solution comprising the listed features.

In the present invention, the expressions "preferably", "preferentially", "more preferably", and "appropriately" are solely used for describing better embodiments or examples, and it should be understood that the scope of the present invention is not intended to be limited. In the present invention, the expressions "optionally", "optional" and "alternative" mean that the subject modified by the expressions are dispensable, that is, the expressions mean that the parallel technical solutions "with" or "without" the subject modified by the expressions can both be selected. If the expression "alternative" appears multiple times in a technical solution, unless otherwise specified and without any contradictions or mutually restrictive relationships, the expression "alternative" is independent in each occurrence.

All documents mentioned in the present invention are incorporated in the present application by reference as if each document is cited separately as a reference. The cited documents involved in the present invention are incorporated by reference in their entireties for all purposes unless they conflict with the objectives and/or technical solutions of the present application. When the present invention relates to a cited document, definitions of relevant technical features, terms, nouns, phrases, etc. in the cited document are also incorporated herein. When the present invention relates to a cited document, examples and preferred modes of the related technical features cited are also incorporated by reference in the present application but are limited to those that enable the implementation of the present invention. It should be understood that where a reference conflicts with the description of the present application, the present application shall prevail, or the reference should be modified as appropriate based on the description of the present application.

In a first aspect, the present invention relates to a plasmid, wherein the plasmid comprises an editable region of two prokaryotic telomerase target sequences which are connected in tandem in the same direction, and both ends of the editing region are independently provided with one or more restriction endonuclease digestion sites.

In some embodiments, the length of the plasmid is ≤ 2500 bp, e.g., 2400 bp, 2300 bp, 2200 bp, 2100 bp, 2050 bp, 2000 bp, 1950 bp, 1900 bp, 1850 bp, 1800 bp, or less.

In some embodiments, the plasmid further comprises a prokaryotic replicon and a selectable gene.

Typical selectable genes are, for example, antibiotic resistance genes, such as kanamycin resistance gene (KanR), tetracycline resistance gene (tetR), and neomycin phosphotransferase gene (npt). The selectable gene may also be a gene of an enzyme for compound detoxification, a selectable marker gene for carbohydratemetabolizing enzyme, etc.

In some embodiments, the number of restriction endonuclease digestion site at each end is independently selected from 1, 2, 3, 4, 5, 6, or more, and preferably, the types of the restriction endonuclease digestion site at the same end are different from each other.

In a more preferred embodiment, the restriction endonuclease digestion sites located at the same end of the editable region overlap.

In a more preferred embodiment, all the restriction endonuclease digestion sites are different from each other.

The restriction endonuclease digestion site at each end may be selected from any one or more of: digestion sites of AccI, AflII, AgeI, Apal, AscI, AvrII, BamHI, BglI, BglII, BsaI, BspQI, BstBI, BsteII, ClaI, EcoNI, EcoRI, EcoRV, FseI, HindIII, KpnI, MfeI, MluI, NcoI, NdeI, NheI, NotI, PacI, PstI, PvuI, PvuII, SacI, SacII, SalI, ScaI, SmaI, SpeI, StuI, SwaI, XbaI, XhoI, and XmaI, preferably from any one or more of digestion sites of HindIII, SalI, SmaI, BamHI, EcoRV, XbaI, KpnI, and EcoRI. In a specific embodiment, the recognition sites of HindIII, SalI, SmaI, and BamHI are inserted near one end of the editable region, and the recognition sites of EcoRV, XbaI, KpnI, and EcoRI are inserted near the other end of the editable region, so that 16 groups of alternative digestion combinations can be provided. Thus, the practicability and broad-spectrum applicability are better.

According to the present invention, a closed linear DNA molecule is generated by the action of the prokaryotic telomerase on a DNA amplified from a DNA template containing at least one prokaryotic telomerase target sequence. The prokaryotic telomerase target sequence is any DNA sequence existing in a DNA template, that enables it to be transformed into a closed linear DNA through the enzymatic activity of the prokaryotic telomerase. In other words, the prokaryotic telomerase target sequence is necessary for cleavage by the prokaryotic telomerase followed by linkage of a double-stranded DNA to form a covalently closed linear DNA.

Prokaryotic telomerases and target sequences thereof are well known to those skilled in the art, and are, for example, prokaryotic telomerases and their target sequences described in CN 102301010 A published on December 28, 2011. Typically, the prokaryotic telomerase target sequence includes any complete palindromic sequence, that is, any double-stranded DNA sequence with two-fold rotational symmetry. In *Borrelia burgdorferi,* the complete inverted repeat sequence has a length of 14 base pairs. In various mesophilic phages, the complete inverted repeat sequence has a length of 22 base pairs or more. In addition, in some cases, for example, as for *E. coli* N15, a central complete inverted palindromic sequence is flanked by an inverted repeat sequence to form a part of a larger incomplete inverted palindromic sequence. In some embodiments, the prokaryotic telomerase target sequence is derived from *E. coli* N15 phage, *Klebsiella* Phi K02 phage, *Yersinia* Py54 phage, *Halomonas* Phi HAP phage, *Vibrio* VP882 phage, or *Borrelia burgdorferi* lpB31.16, or any variant thereof. Preferably, the prokaryotic telomerase target sequence is derived from phage N15 TelN or a variant thereof. In some specific embodiments, the prokaryotic telomerase target sequence comprises a sequence set forth in SEQ ID NO: 1, or a nucleotide sequence that has at least 80% identity with the sequence set forth in SEQ ID NO: 1 and is capable of functioning as a telomerase, for example, a nucleotide sequence that has at least 85%, 90%, 95%, 96%, 97%, 98%, or 99% identity, for example in which truncation, insertion, substitution and/or deletion of one or several nucleotides have occurred.

The plasmid can be obtained by engineering a conventional cloning vector, such as a prokaryotic vector, preferably an *Escherichia coli* plasmid vector, more preferably a plasmid smaller than 2000 bp. In some specific embodiments, it is engineered from pUC57-Kan-mini (1824bp). In some embodiments, the plasmid comprises a nucleotide sequence that has at least 80% identity with the sequence set forth in SEQ ID NO: 2, for example, a nucleotide sequence that has at least 85%, 90%, 95%, 96%, 97%, 98%, or 99% identity, for example in which truncation, insertion, substitution and/or deletion of one or several nucleotides have occurred. In some specific embodiments, the plasmid comprises a nucleotide sequence set forth in SEQ ID NO: 2.

In the editable region, in some embodiments, the two prokaryotic telomerase target sequences connected in tandem in the same direction are directly connected; and in some embodiments, a nucleotide sequence less than 30 bp, or less than 20 bp, or less than 10 bp is further comprised between the two prokaryotic telomerase target sequences connected in tandem in the same direction, and the nucleotide sequence is preferably a site or related sequence facilitating the insertion of the gene of interest.

In a second aspect, the present invention relates to a recombinant plasmid, wherein a gene of interest is inserted between the two prokaryotic telomerase target sequences of the plasmid as described above.

In some embodiments, the expression product of the gene of interest is an interfering RNA or an aptamer.

The interfering RNA can be selected from, for example, an siRNA, an shRNA, or an miRNA.

In some embodiments, the expression product of the gene of interest is an mRNA, and such an mRNA is preferably capable of expressing a protein, such as a protein that endows the target cell with certain required characteristics, e.g., a fluorescent protein that allows for cell tracing, and an active enzyme provided to compensate for losses or alterations in the target cell.

The gene of interest may include, for example, a gene (nucleotide sequence) encoding a protein that is defective or absent in a recipient individual or target cell; a gene that encodes a protein having a desired biological or therapeutic effect (such as antibacterial, antiviral, or antitumor/anticancer function); a nucleotide sequence encoding an RNA that inhibits or reduces the production of a deleterious or otherwise undesirable protein (for example, a nucleotide sequence encoding an RNA interfering agent as defined above); and/or a nucleotide sequence encoding an antigenic protein.

Suitable genes of interest include, but are not limited to, nucleic acids encoding proteins for the treatment of: endocrine, metabolic, hematologic, cardiovascular, neurological, musculoskeletal, urological, pulmonary and immune disorders, including, for example, cancers, inflammatory disorders, immune disorders, and chronic and infectious disorders. The cancers may be, for example, tumors arising from lesions in any of bone, bone junctions, muscle, lung, trachea, heart, spleen, arteries, veins, blood, capillaries, lymph nodes, lymphatic vessels, lymph fluid, oral cavity, pharynx, esophagus, stomach, duodenum, small intestine, colon, rectum, anus, appendix, liver, gallbladder, pancreas, parotid gland, sublingual gland, urinary system, kidney, ureter, bladder, urethra, ovary, fallopian tube, uterus, vagina, vulva, scrotum, testis, vas deferens, penis, eye, ear, nose, tongue, skin, brain, brain stem, medulla oblongata, spinal cord, cerebrospinal fluid, nerve, thyroid, parathyroid, adrenal gland, hypophysis, pineal gland, pancreatic islets, thymus, gonads, sublingual glands and parotid glands; the immune disorders may be, for example, systemic lupus erythematosus, multiple sclerosis, type I diabetes, psoriasis, ulcerative colitis, Sjogren syndrome, scleroderma, polymyositis, rheumatoid arthritis, mixed connective tissue disease, primary biliary cirrhosis, autoimmune hemolytic anemia, Hashimoto thyroiditis, Addison's disease, vitiligo, Graves' disease, autoimmune myasthenia gravis, ankylosing spondylitis, allergic osteoarthritis, hypersensitivity angiitis, autoimmune neutropenia, idiopathic thrombocytopenic purpura, lupus nephritis, chronic atrophic gastritis, autoimmune infertility, endometriosis, Pasture's disease, pemphigus, discoid lupus erythematosus or dense deposit disease; and the infectious disorders may be any one or any combination of a viral, bacterial, fungal and parasitic infection.

Suitable genes of interest include, but are not limited to, those that encode at least one of the following various proteins: interferons (e.g., IFN-γ, IFN-α, IFN-β, IFN-ω, and IFN-τ); insulin (e.g., Novolin, Humulin, Humalog, Humalog, and Lantus); erythropoietin ("EPO", e.g., epoetin-α, darbepoetin-α, and epoetin-β); antibodies (e.g., monoclonal antibodies, such as rituximab, infliximab, trastuzumab, HumiraTM (adalimumab), omalizumab, tositumomab, RaptivaTM (efalizumab), ErbituxTM (cetuximab), and bevacizumab); antigen-binding fragments, including monoclonal antibodies (e.g., ranibizumab); blood factors (e.g., alteplase, tissue plasminogen activator protein, recombinant human factor VIIa, factor VIIa, factor VIII, factor IX, β-globulin, and hemoglobin); colony stimulating factors (e.g., filgrastim (G-CSF), Neulasta (PEGylated filgrastim), granulocyte colony stimulating factor (G-CSF), granulocyte monocyte colony stimulating factor, macrophage colony stimulating factor, and megakaryocyte colony stimulating factor); growth hormones (e.g., somatotropin and growth hormone); interleukins (e.g., IL-1, IL-2, IL-3, IL-4, IL-5, IL-6, IL-7, IL-8, and IL-9); growth factors (e.g., beclapermin (PDGF), trafermin (bFGF), ancestim (stem cell factor), keratinocyte growth factor, acidic fibroblast growth factor, stem cell factor, basic fibroblast growth factor, and hepatocyte growth factor); soluble receptors (e.g., soluble TNF-α-binding receptors, such as etanercept, soluble VEGF receptors, soluble interleukin receptors, and soluble γ/δT cell receptors); enzymes (e.g., α-glucosidase, β-glucocerebrosidase, and alglucerase), and enzyme activators (e.g., tissue plasminogen activator); chemokines (e.g., IP-10, Mig, Groα/IL-8, RANTES, MIP-1α, MIP-1β, MCP-1, and PF-4); angiogenic agents (e.g., vascular endothelial growth factor (VEGF)); antiangiogenic agents (e.g., soluble VEGF receptors); protein vaccines; neuroactive peptides, such as bradykinin, cholecystokinin, gastrin, secretin, oxytocin, gonadotropin-releasing hormone, β-endorphin, enkephalin, substance P, growth hormone release inhibitory factor, prolactin, galanin, growth hormone releasing hormone, bombesin, dynorphin, neurotensin, motilin, thyrotropin, neuropeptide Y, luteinizing hormone, calcitonin, insulin, glucagon, vasopressin, angiotensin II, thyrotropin releasing hormone, vasoactive intestinal peptide, and sleep-inducing peptide; other proteins, e.g., thrombolytic agents, atrial natriuretic peptide, bone morphogenetic protein, thrombopoietin, relaxin, glial fibrillary acidic protein, follicle-stimulating hormone, human α-1 antitrypsin, leukemia inhibitory factor, transforming growth factor, insulin-like growth factor, luteinizing hormone, macrophage activating factor, tumor necrosis factor, neutrophil chemotactic factor, nerve growth factor, and tissue inhibitors of metalloproteinases; vasoactive intestinal peptide, angiopoietin, angiopoietin, and fibrin; hirudin; leukemia inhibitory factor; IL-1 receptor antagonists (e.g., anakinra); ion channels, e.g., cystic fibrosis transmembrane conductance regulator protein (CFTR); dystrophin; utrophin (a tumor inhibitor); lysosomal acid α-glucosidase (GAA); *etc.* Suitable genes of interest also include those encoding functional fragments of any of the above proteins; and nucleic acids encoding functional variants of any of the above proteins.

In some embodiments, the gene of interest may be inserted into the plasmid in the form of an expression cassette.

It is readily understood that the plasmid/recombinant plasmid may also comprise at least one of the following elements: a promoter, an enhancer, an internal ribosome entry site, a reporter gene, a conjugated marker gene, and a transcription termination signal. These elements are preferably located in the expression cassette of the gene of interest.

A promoter is a nucleotide sequence that initiates and regulates transcription of a polynucleotide. The promoter includes an inducible promoter (the expression of a polynucleotide sequence operably linked to the promoter is induced by an analyte, a cofactor, a regulatory protein, etc.), a repressible promoter (the expression of a polynucleotide sequence operably linked to the promoter is repressed by an analyte, a cofactor, a regulatory protein, etc.), and a constitutive promoter. The term "promoter" is intended to include full-length promoter regions and their functional fragments (e.g., for controlling transcription or translation). The promoter preferably includes a promoter that initiates prokaryotic expression. However, where a gene of interest is present, a promoter for controlling the expression of the gene of interest may also be comprised, which may be a non-cell-specific promoter; or a "cell-specific", "cell type-specific", "cell lineage-specific", or "tissue-specific" promoter depending on the desired administration site of the product of the gene of interest, which allow for respective expressions in limited cell types and tissue types.

Typical reporter genes are, for example, fluorescent protein genes, luciferase genes, and LacZ. Both fluorescent proteins and luciferases belong to luminescent proteins, and the fluorescence expression can be detected by a camera or a similar device. A fluorescent protein works by absorbing light of one color (excitation) and then emitting lower-energy light of a different color (emission). On the contrary, luciferases (and other bioluminescent enzymes) emit light by catalyzing the chemical reaction of a substrate (i.e., a fluorescein). Unlike the above two markers, LacZ does not emit light. The product of LacZ gene, β-galactosidase, can catalyze the transformation of X-gal into an opaque blue compound similar to indigo. The fluorescent protein is, for example, a green fluorescent protein, a blue fluorescent protein, a yellow fluorescent protein, an orange fluorescent protein, or a red fluorescent protein. The green fluorescent protein may be either a common GFP or an engineered GFP gene, such as an enhanced GFP gene (EGFP); the blue fluorescent protein may be selected from EBFP, Azuritc, TagBFP, etc.; the yellow fluorescent protein may be selected from EYFP, Ypct, PhiYFP, etc.; the orange fluorescent protein may be selected from mKO, mOrange, mBanana, etc.; and the red fluorescent protein may be selected from TagRFP, mRuby, mCherry, and mKatc.

A typical conjugated marker gene is, for example, a gene capable of expressing biotin or a derivative thereof, wherein the derivative of biotin is any one of D-biotin, activated biotin, biocytin, ethylenediamine-biotin, cadaverine-biotin, and desthiobiotin.

An internal ribosome entry site (IRES) is a genetic engineering element with hundreds of base pairs (bp) in length. The IRES was first found in viruses, and it was later found that some eukaryotic cells also have IRES elements on their mRNAs. The IRES can guide a ribosome to enter the sequence and translate an open reading frame in the 3' direction. Where an IRES sequence is inserted between two open reading frames (ORFs) of the same transcript, the one transcript can be translated into two independent proteins.

A transcription termination signal refers to a stem-loop structure formed by a section of RNA sequence produced during transcription, which can be specifically recognized by the RNA polymerase transcription complex to terminate transcription.

In some embodiments, the gene of interest is a nucleic acid vaccine.

In a third aspect, the present invention relates to a method for preparing a linear closed DNA, comprising:
a) digesting a recombinant plasmid comprising a gene of interest with a prokaryotic telomerase to separate the gene of interest from a heterogeneous nucleic acid in the recombinant plasmid;
b) using at least two restriction endonucleases for digestion such that the ends of the heterogeneous nucleic acid are exposed, and removing the heterogeneous nucleic acid by using an exonuclease; and
c) separating and purifying the linear closed DNA containing the gene of interest;
wherein the recombinant plasmid comprises prokaryotic telomerase target sequences and restriction endonuclease digestion sites, and the gene of interest is inserted between the prokaryotic telomerase target sequences.

A heterogeneous nucleic acid refers to an unnecessary or additional sequence other than the gene of interest. Such unnecessary or additional sequences typically include various elements in the plasmid as described above, such as replicons and selectable genes. However, it is easy to understand that the heterogeneous nucleic acid in the present invention refers to the vast majority, but not all, of the above unnecessary or additional sequences. For example, at least the prokaryotic telomerase target sequences (such as telL and telR) directly connected to the gene of interest cannot be removed.

In some embodiments, the recombinant plasmid is the recombinant plasmid as defined above, and the content defined in the first and second aspects of the present invention now also applies to the third aspect.

In some embodiments, the added amount of the prokaryotic telomerase is 8000-15000 U/mg, e.g., 9000 U/mg, 10000 U/mg, 11000 U/mg, 12000 U/mg, 13000 U/mg, and 14000 U/mg.

In some embodiments, the digestion temperature of the prokaryotic telomerase is 25-35°C, preferably 27-33°C.

In some embodiments, the digestion time of the prokaryotic telomerase is 1-8 hours, e.g., 2, 3, 4, 5, 6, and 7 hours.

In some embodiments, the added amount of each restriction endonuclease is independently selected from 1000-6000 U/mg, e.g., 2000 U/mg, 3000 U/mg, 4000 U/mg, and 5000 U/mg.

In some embodiments, the digestion temperature of the restriction endonuclease is 32-42°C, preferably 34-40°C, e.g., 35°C, 36°C, 37°C, 38°C, and 39°C.

In some embodiments, the digestion time of the restriction endonuclease is 1-4 hours, e.g., 2 hours and 3 hours.

In some embodiments, the exonuclease includes Exonuclease III and/or T5 Exonuclease.

In some embodiments, the added amount of the exonuclease is 3000-6000 U/mg, e.g., 3500 U/mg, 4000 U/mg, 4500 U/mg, 5000 U/mg, and 5500 U/mg.

In some embodiments, the digestion temperature of the exonuclease is 34-40°C, e.g., 35°C, 36°C, 37°C, 38°C, and 39°C.

In some embodiments, the digestion time of the exonuclease is 2-12 hours, e.g., 3, 4, 5, 6, 7, 8, 9, 10, 11 hours, more preferably 3-6 hours.

In some embodiments, after the digestion with the exonuclease, EDTA with a final concentration of 1-50 mM, e.g., 5 mM, 10 mM, 15 mM, 20 mM, 25 mM, 30 mM, 35 mM, 40 mM, and 45 mM, is added.

In some embodiments, after the digestion with the exonuclease, EDTA is added, and the temperature is raised to 70-80°C for 5-30 minutes, e.g., 10, 15, 20, or 25 minutes, of treatment.

After the digestion with the exonuclease, both EDTA and high-temperature methods are used to inactivate the enzymes, thereby effectively inhibiting the residual activity of the endonuclease and exonuclease in the system and greatly improving stability during large-scale production.

In some embodiments, after finishing the digestion with the prokaryotic telomerase and/or the digestion with the restriction endonuclease, the temperature is raised to 70-80°C for 5-30 minutes, e.g., 10, 15, 20, or 25 minutes, of treatment.

In some embodiments, the separation and purification method comprises one or any combination of gel filtration chromatography, anion exchange chromatography, concentration and liquid exchange, and sterilization filtration.

In some embodiments, the separation and purification method comprises sequentially gel filtration chromatography and anion exchange chromatography. In some other embodiments, the separation and purification method comprises sequentially gel filtration chromatography, anion exchange chromatography, and concentration and liquid exchange.

LcDNAs are purified by a process of gel filtration chromatography combined with anion exchange chromatography, which not only improves the purity and quality of the LcDNA product, but is also more compliant; in addition, this process can significantly improve the product yield on the premise of ensuring purity, and thus it is more suitable for commercial use.

In some embodiments, a packing for the gel filtration chromatography is Bestarose 6FF, and an eluate from the first ultraviolet peak with an absorbance of ≥ 50 mAu is collected.

In some embodiments, a packing for the anion exchange chromatography is Fractogel EMD DEAE.

In some embodiments, loading conditions for the anion exchange chromatography comprise:
using a loading solution containing 30%-70% (e.g., 40%, 50%, or 60%, in percentage by volume) chromatographic solution B, with a loading capacity of 0.20-1.0 mg/mL (e.g., 0.25 mg/mL, 0.3 mg/mL, 0.35 mg/mL, 0.4 mg/mL, 0.45 mg/mL, 0.5 mg/mL, 0.6mg/mL, 0.7 mg/mL, 0.8 mg/mL, or 0.9 mg/mL) and a loading flow rate of ≤ 120 cm/h, wherein
the chromatographic solution B comprises 0.5-1.5 M NaCl, 5-15 mM EDTA, and a buffer substance, pH 7.2-7.8.

As used herein, the term "buffer substance" refers to an aqueous solution or composition that resists pH changes when an acid or a base is added to the solution or composition. This resistance to pH changes is due to the buffering properties of such solutions. Therefore, a solution or composition showing buffering activity is referred to as a buffer or a buffer solution. The buffer that can be used in the method of the present invention is preferably selected from a phosphate buffer, a phosphate buffered saline buffer (PBS), 2-amino-2-hydroxymethyl-1,3-propanediol (TRIS) buffer, a TRIS buffered saline solution (TBS), TRIS/EDTA (TE), etc.

In some preferred embodiments, loading conditions for the anion exchange chromatography comprise:
using a loading solution containing 50% (in percentage by volume) chromatographic solution B, with a loading capacity of 0.20-1.0 mg/mL (e.g., 0.25 mg/mL, 0.3 mg/mL, 0.35 mg/mL, 0.4 mg/mL, 0.45 mg/mL, 0.5 mg/mL, 0.6 mg/mL, 0.7 mg/mL, 0.8 mg/mL, or 0.9 mg/mL) and a loading flow rate of ≤ 120 cm/h, wherein
the chromatographic solution B comprises 0.5-1.5 M NaCl, 5-15 mM EDTA, and 50-150 mM Tris-HCl, pH 7.2-7.8.

More preferably, the chromatographic solution B comprises 1 M NaCl, 10 mM EDTA, and 100 mM Tris-HCl, pH 7.5.

In some embodiments, elution conditions for the anion exchange chromatography comprise:
elution with 65%-100% (e.g., 70%, 80%, or 90%, in percentage by volume) chromatographic solution B at an elution flow rate of 30-120 cm/h (e.g., 40 cm/h, 50 cm/h, 60 cm/h, 70 cm/h, 80 cm/h, 90 cm/h, 100 cm/h, or 110 cm/h), and collection of an eluate with an absorbance of ≥ 100 mAu.

In some preferred embodiments, elution conditions for the anion exchange chromatography comprise:
elution with 80% (in percentage by volume) chromatographic solution B at an elution flow rate of 30-120 cm/h (e.g., 40 cm/h, 50 cm/h, 60 cm/h, 70 cm/h, 80 cm/h, 90 cm/h, 100 cm/h, or 110 cm/h), and collection of an eluate with an absorbance of ≥ 100 mAu.

In some embodiments, the concentration and liquid exchange method is tangential flow filtration.

In a fourth aspect, the present invention relates to a kit comprising a prokaryotic telomerase and the plasmid as described above;
or comprising a prokaryotic telomerase and the recombinant plasmid as described above.

In some embodiments, the kit further comprises a restriction endonuclease and/or an exonuclease, wherein the restriction endonuclease is capable of recognizing the restriction endonuclease digestion site.

The kit may further comprise conventional components such as nucleic acidfree water and an instruction for use.

In a fifth aspect, the present invention provides a linear closed DNA obtained by the above preparation method. The linear closed DNA comprises a gene of interest.

The present invention further provides a composition comprising the above linear closed DNA. In some embodiments, the composition comprises the above linear closed DNA with a purity of at least 90%. In some preferred embodiments, the composition comprises the above linear closed DNA with a purity of at least 99%.

The description of the first and second aspects of the present invention also applies to the fourth and fifth aspects.

The embodiments of the present invention will be described in detail below with reference to the examples. It should be understood that these examples are merely intended to illustrate the present invention but not to limit the scope of the present invention. The experimental methods in the following examples, where no specific conditions are specified, can be performed preferentially according to the instructions provided in the present invention, experimental manual or conventional conditions in the art, other experimental methods known in the art, or the conditions suggested by the manufacturer.

In the following specific examples, the measurement parameters related to the components of the raw materials may have slight deviations within the weighing precision, unless otherwise specified. When temperature and time parameters are involved, acceptable deviations due to instrument testing precision or operating precision are allowed.

### Example 1 The design of a universal circular plasmid vector, CFL universal, for LcDNA production

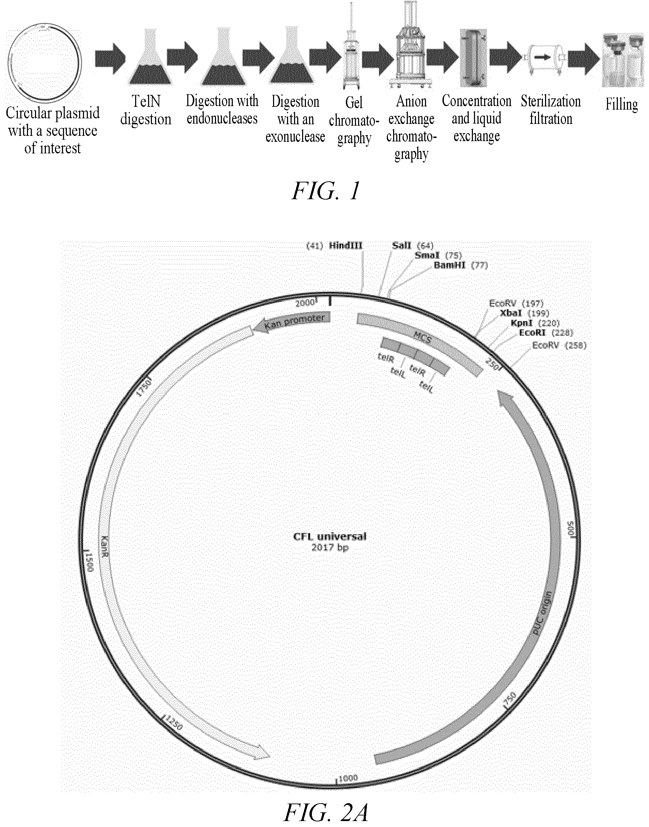

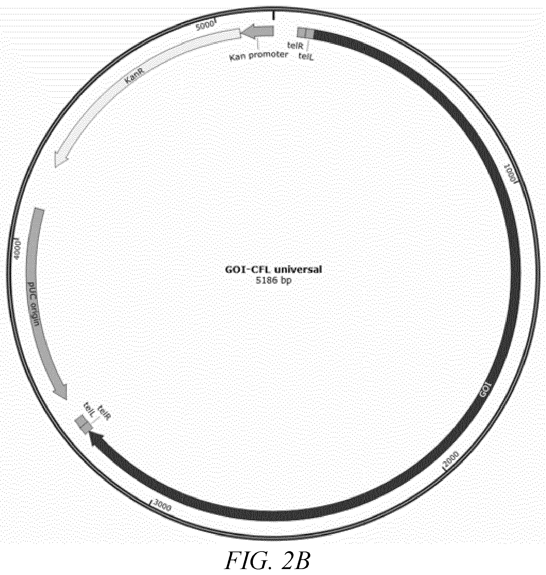

By designing a universal LcDNA template plasmid backbone, CFL universal, to make the size of the template plasmid backbone minimized, the theoretical recovery rate of LcDNAs was improved from the source. The specific design method was as follows: firstly, two TelN enzyme recognition sequences, each of which was TelRL (56 bp, see SEQ ID NO: 1 for sequence information), were successively inserted into pUC57-Kan-mini (1824 bp), and the recognition sites of HindIII, SalI, SmaI, and BamHI were then successively inserted at one end near the prokaryotic telomerase TelN recognition site while the recognition sites of EcoRV, XbaI, KpnI, and EcoRI were inserted at the other end near the TelN recognition site, which provided 16 groups of alternative digestion combinations for restriction endonuclease digestion in the second step, greatly improving the practicability and broad-spectrum applicability of CFL Universal. The length of the engineered CFL Universal was 2017 bp. It was only necessary to design the sequence of interest to be inserted between the two TelN recognition sites, each of which was TelRL, in CFL universal. The map is shown in FIG. 2A (see SEQ ID NO: 2 for the sequence information), in which a gene of interest (GOI) was inserted with a length of 3169 bp as an example, as shown in FIG. 2B. The compliant synthesis of designed circular plasmid sequence was entrusted to Nanjing GenScript Biotech Co., Ltd., and then after successively *Escherichia coli* library construction, high-density fermentation, cell lysis, clarification and filtration, concentration and incubation, chromatographic purification, concentration and liquid exchange, sterilization filtration, and filling, a circular plasmid with a high purity and a high quality was obtained as the starting material for LcDNA production.

### Example 2 Digestion and purification of LcDNAs

### (1) Digestion of LcDNAs

LcDNA digestion involved three steps, i.e., TelN digestion, exonuclease digestion, and endonuclease digestion successively. When the vector CFL universal was selected as the backbone of the circular plasmid as a starting material, the parameters of the TelN digestion process were as follows: for the enzyme TelN, the added amount was 10000 U/mg, the digestion temperature was 30°C, the digestion time was 3 hours, the digestion termination temperature was 75°C, and the treatment time was 15 minutes. The parameters of the endonuclease digestion process were as follows: endonucleases that did not have the corresponding recognition sites for the inserted sequence of interest were selected, e.g., HindIII, SalI, SmaI, BamHI, EcoRV, XbaI, KpnI, and EcoRI, and two endonucleases were added, with the added amount of each endonuclease being 3000 U/mg, the digestion temperature being 37°C, the digestion time being 2 hours, the digestion termination temperature being 75°C, and the treatment time being 15 minutes. The parameters of the exonuclease digestion process were as follows: the exonuclease was Exo III or T5 Exo, the added amount was 4500 U/mg, the digestion temperature was 37°C, and the digestion time was 4 hours. At the end of digestion, EDTA with a final concentration of 15 mM was first added for treatment at 75°C for a time of 10-20 minutes.

### (2) Gel filtration chromatography (GF) of LcDNAs

After the three-step digestion of LcDNAs, the reaction liquid of digestion was treated by centrifugation or filtration, followed by gel filtration chromatography. The parameters of the gel filtration chromatography process were as follows: the packing was Bestarose 6FF, the height of the chromatographic column was ≥ 20 cm, the symmetry factor was 0.8-1.8, and the plate number was > 2000. The loading volume was ≤ 0.3 column volumes, the loading flow rate was 60 cm/h, the elution flow rate was ≤ 120 cm/h, and the equilibrium and elution buffer was GF Buffer A (the composition was 0.5 M NaCl, 5 mM EDTA, and 50 mM Tris-HCl). An eluate from the first ultraviolet peak with an absorbance of ≥ 50 mAu was collected.

### (3) Anion exchange chromatography (AEX) of LcDNAs

After gel filtration chromatography, the collected eluate was subjected to anion exchange chromatography. The parameters of the anion exchange chromatography process were as follows: the packing was Fractogel EMD DEAE, the height of the chromatographic column was ≤ 20 cm, the symmetry factor was 0.8-1.8, and the plate number was > 2000. The loading conditions were a loading solution containing 30%-70% (in percentage by volume) chromatographic solution B, the loading capacity was 0.25-1.0 mg/mL, the loading flow rate was ≤ 120 cm/h, and the retention time was ≥ 6 minutes. The elution flow rate was 30-120 cm/h, and an eluate with an absorbance of ≥ 100 mAu was collected. The composition of chromatographic solution A was 10 mM EDTA and 100 mM Tris-HCl, pH 7.5, and the composition of chromatographic solution B was 1 M NaCl, 10 mM EDTA, and 100 mM Tris-HCl, pH 7.5. Equilibrium with 100% chromatographic solution A, washing off heterogeneous materials with 58% chromatographic solution B, and elution with 65%-100% chromatographic solution B were carried out. For the optimization of elution conditions, as shown in FIG. 8, the elution with chromatographic solution B in a gradient of 0-100% was used, wherein the maximum ultraviolet peak appeared in the chromatogram upon elution with 67.5% chromatographic solution B. In the case of elution with 80% chromatographic solution B, the data of optimization of the anion exchange chromatography process are shown in Tables 1A and 1B, and the corresponding elution curves are shown in FIGs. 6A, 6B, 7A, and 7B.
Table 1 Summary table of data of optimization of anion exchange chromatography for LcDNAs

**Table 1A Optimization of loading conditions**

| Loading condition | Loading amount (mg) | Elution amount (mg) | % yield |
|---|---|---|---|
| 30% chromatographic solution B | 0.787 | 0.534 | 67.85 |
| 50% chromatographic solution B | 1.05 | 0.853 | 81.27 |
| 70% chromatographic solution B | 0.787 | 0.044 | 5.62 |

**Table 1B Optimization of loading capacities**

| Loading amount (mg) | Loading capacity, mg/mL | Elution amount (mg) | % yield |
|---|---|---|---|
| 1.25 | 0.25 | 1.08 | 86.49 |
| 2.50 | 0.50 | 2.03 | 81.21 |
| 5.00 | 1.00 | 3.83 | 76.57 |

### (4) LcDNA concentration and liquid exchange

After finishing the anion exchange chromatography, the collected eluate was subjected to sample concentration by a tangential flow filtration method to a dialysis point of 1.0 mg/mL ± 0.3 mg/mL, followed by liquid exchange with a final storage buffer such as Tris-EDTA with a volume of 10 ± 1 times the sample volume. Membrane packages for tangential flow filtration had a pore size with a molecular weight cutoff of 10 kD, with specifications of 50 cm², 0.01 m², 0.1 m², and 0.5 m², and a membrane package with appropriate specifications was selected according to the production scale.

### (5) Sterilization and filtration of LcDNAs

An intermediate sample of LcDNAs after the concentration and liquid exchange was subjected to sterilization filtration operation. The sterilization filter had a pore size of 0.22 µm, with the specifications being a syringe filter, a 1L filter cup, and a capsule filter. A filter with appropriate specifications was selected according to the volume of the sample after concentration and liquid exchange.

### (6) LcDNA filling and storage

After sterilization filtration, the LcDNA sample was subjected to filling in an ultra-clean bench or isolator according to the split packaging specifications of the project. After filling, the finished product of LcDNAs was stored in an ultra-low temperature refrigerator at -70°C ± 10°C.

### Example 3 Quality detection of LcDNAs

The length of the exemplary LcDNA is 3169 bp, and the complete sequence map of the circular plasmid in which the LcDNA is inserted is shown in FIG. 2B. The intermediate samples and finished products of LcDNAs were subjected to quality detection. The detection method and the corresponding results were as follows:

### 3.1 Detection of intermediate samples and finished products of LcDNAs by agarose gel electrophoresis (AGE)

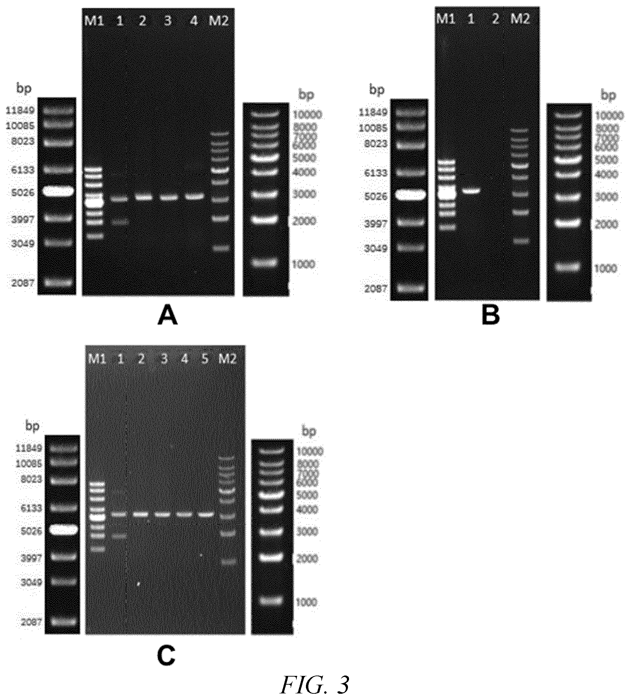

The intermediate samples and finished products of LcDNAs were detected by using 0.7% agarose gel, with 1×TAE as an electrophoresis buffer. The electrophoresis voltage was 150 V, and the electrophoresis time was 30 minutes. After finishing the electrophoresis, an image was taken using Image Lab software. The AGE detection results are shown in FIG. 3 and Table 2.

**Table 2 Summary table of LcDNA production data**

| Amount of digested circular plasmid samples | Batch No. | % GF chromatography yield | % AEX chromatography yield | % total yield of LcDNAs |
|---|---|---|---|---|
| 60 mg | 1 | 47.63 | 79.40 | 30.10 |
| | 2 | 50.44 | 75.34 | 33.10 |
| 100 mg | 3 | 46.97 | 79.93 | 30.30 |
| 150 mg | 4 | 47.92 | 68.57 | 28.90 |
| | 5 | 39.37 | 75.10 | 25.90 |

| | | | | |
|---|---|---|---|---|
| Note: Total yield = Amount of finished products of LcDNAs / Amount of digested circular plasmid samples × 100% | | | | |

Compared with small-scale production methods, after the circular plasmid underwent digestion with TelN, separation by agarose gel electrophoresis (AGE), gel cutting, and purification by Axygen gel extraction kit (AXYGEN, catalog no.: UE-GX-50), the LcDNA yield was low, less than 10%, and the quality did not meet clinical or commercial requirements. After fermentation using engineering bacteria capable of producing LcDNAs, followed by one-step Q anion exchange chromatography (NanoGel-50Q, Suzhou NanoMicro Technology Co., Ltd., catalog no.: 04064-050200-2100) purification method, the LcDNA yield was low, less than 10%, and the LcDNA purity was not high, which did not meet clinical or commercial requirements.

### 3.2 Detection of LcDNA purity by high-performance liquid chromatography (IEC-HPLC)

Using Agilent 1260 high-performance liquid chromatograph, the purity of the finished products of LcDNAs was detected, and the detection results were summarized in Table 3.

### 3.3 Verification of LcDNAs by Sanger sequencing

Appropriate sequencing primers were designed according to the inserted sequence of interest, and the LcDNA sample was subjected to Sanger sequencing verification of the whole plasmid. The sequencing results were summarized in Table 3.

### 3.4 Nanorange fluorescence detection of total residual protein in LcDNAs

Total residual protein in finished products of LcDNAs were detected by NanoOrange Kit (ThermoFisher, catalog no.: N10271), and values were read by a microplate reader. The detection results were summarized in Table 3.

### 3.5 Detection of residual host DNAs (from E. coli) in LcDNAs by the fluorescence quantitative PCR method (qPCR)

The residual host DNAs (from *E. coli*) in LcDNAs were detected using a realtime fluorescence quantitative PCR instrument (ThermoFisher, model: 7500) and residual *E. coli* DNA detection kit (Huzhou Shenke Biotechnology Co., Ltd., catalog no.: SK030202E100), and the detection results were summarized in Table 3.

### 3.6 Detection of bacterial endotoxin in LcDNAs by gel-clot test method (Limulus Amebocyte Lysate, LAL)

Bacterial endotoxin in LcDNAs was detected by gel-clot test method using LAL, and the results were summarized in Table 3.

### 3.7 Direct inoculation method to detect whether LcDNAs were sterile

The LcDNA sample was pipetted by a disposable sterile syringe or sterilized pipette, inoculated into 100 mL of a thioglycolate FTM culture medium, and cultured at 30-35°C for 14 days, and it was detected whether the LcDNA sample was sterile. The detection results were summarized in Table 3.

**Table 3 Summary table of detection results of finished products of LcDNAs**

| LcDNA quality standard | | | | Detection results of 5 batches of LcDNAs | | | | |
|---|---|---|---|---|---|---|---|---|
| No. | Key quality attributes | Methods | Standard requirem ents | 1 | 2 | 3 | 4 | 5 |
| 1 | Appearanc e | Visual observation method | Colorless clear liquid | Colorle ss and clear | Colorle ss and clear | Colorle ss and clear | Colorle ss and clear | Colorle ss and clear |
| 2 | DNA purity | Ultraviolet spectrophot ometry (260 nm/280 nm) | 1.8-2.0 | 1.85 | 1.9 | 1.9 | 1.9 | 1.8 |
| 3 | Concentrat ions | Ultraviolet spectrophot ometry (260 nm) | 1.0 mg/mL ± 0.1 | 1.0 | 1.0 | 1.0 | 1.1 | 1.0 |
| 4 | pH | pH meter | 7.0-8.5 | 7.6 | 7.6 | 7.8 | 7.6 | 7.8 |
| 5 | LcDNA purity | IEC-HPLC | ≥ 90% | 99% | 99% | 100% | 100% | 95% |
| 6 | Whole plasmid sequencing | Sanger | Matching the theoretica l sequence | Consist ent with the theoreti cal sequenc e | Consist ent with the theoreti cal sequenc e | Consist ent with the theoreti cal sequenc e | Consist ent with the theoreti cal sequenc e | Consist ent with the theoreti cal sequenc e |
| 7 | Total residual protein | Nanorange | ≤ 1.0 µg/mL | < 1.0 µg/mL | < 1.0 µg/mL | < 1.0 µg/mL | < 1.0 µg/mL | < 1.0 µg/mL |
| 8 | Residual *Escherichi a coli* host DNAs | qPCR | ≤ 1.0 % | 0.081 | 0.011 | 0.01 | 0.05 | 0.01 |
| 9 | Bacterial endotoxin | TAL | < 10 EU/mg | < 10 EU/mg | < 10 EU/mg | < 10 EU/mg | < 10 EU/mg | < 10 EU/mg |
| 10 | Sterility detection | Culture method | Absence of microbial growth | Absenc e of microbi al growth | Absenc e of microbi al growth | Absenc e of microbi al growth | Absenc e of microbi al growth | Absenc e of microbi al growth |

### Conclusions

1) This LcDNA production process is very stable. The scale of digested circular plasmids is gradually enlarged from 60 mg to 100 mg and to 150 mg, and no abnormal deviation occurs in the production process. This process can also satisfy the need of LcDNA production on the digestion scale of more than 150 mg of the circular plasmids.
2) The total yield of this LcDNA production process is high. As for the 5 batches of production on the three circular plasmid digestion scales, the total yield was as high as 25%-33%.
3) The purity and quality of the finished products of LcDNAs produced by this process can both meet the requirements of the pharmacopoeia, and the finished products of LcDNAs can be used for clinical research and commercial use in gene therapy and other fields.

The above examples merely represent several embodiments of the present invention, giving specifics and details thereof, but should not be understood as limiting the scope of the present patent of disclosure thereby. It should be noted that those of ordinary skill in the art could also make several alterations and improvements without departing from the spirit of the present invention and these would all fall within the scope of protection of the present invention. Therefore, the scope of protection of the present patent of disclosure shall be in accordance with the appended claims, and the description and accompanying drawings can be used to explain the content of the claims.

## Claims

1. A plasmid, **characterized in that** the plasmid comprises an editable region of two prokaryotic telomerase target sequences which are connected in tandem in the same direction, and both ends of the editing regions are independently provided with one or more restriction endonuclease digestion sites.

2. The plasmid according to claim 1, **characterized in that** the plasmid has a length of ≤ 2500 bp, or ≤ 2300 bp, or ≤ 2100 bp.

3. The plasmid according to claim 1, **characterized in that** the plasmid further comprises a prokaryotic replicon and a selectable gene.

4. The plasmid according to claim 3, **characterized in that** the restriction endonuclease digestion site at each end is selected from any one or more of: digestion sites of AccI, AflII, AgeI, ApaI, AscI, AvrII, BamHI, BglI, BglII, BsaI, BspQI, BstBI, BsteII, ClaI, EcoNI, EcoRI, EcoRV, FseI, HindIII, KpnI, MfeI, MluI, NcoI, NdeI, NheI, NotI, PacI, PstI, PvuI, PvuII, SacI, SacII, SalI, ScaI, SmaI, SpeI, StuI, SwaI, XbaI, XhoI, and XmaI, preferably from any one or more of digestion sites of HindIII, SalI, SmaI, BamHI, EcoRV, XbaI, KpnI, and EcoRI.

5. The plasmid according to claim 4, **characterized in that** the digestion sites of HindIII, SalI, SmaI, and BamHI are inserted near one end of the editable region, and the digestion sites of EcoRV, XbaI, KpnI, and EcoRI are inserted near the other end of the editable region.

6. The plasmid according to any one of claims 1-5, **characterized in that** the prokaryotic telomerase target sequence comprises a sequence set forth in SEQ ID NO: 1 or a nucleotide sequence that has at least 80% identity with the sequence set forth in SEQ ID NO: 1 and is capable of functioning as a telomerase.

7. The plasmid according to any one of claims 1-6, **characterized in that** the plasmid comprises a nucleotide sequence that has at least 80% identity with the sequence set forth in SEQ ID NO: 2.

8. A recombinant plasmid, **characterized in that** a gene of interest is inserted between the two prokaryotic telomerase target sequences of the plasmid according to any one of claims 1-7.

9. A method for preparing a linear closed DNA, **characterized by** comprising:
a) digesting a recombinant plasmid comprising a gene of interest with a prokaryotic telomerase to separate the gene of interest from a heterogeneous nucleic acid in the recombinant plasmid;
b) using at least two restriction endonucleases for digestion such that the ends of the heterogeneous nucleic acid are exposed, and removing the heterogeneous nucleic acid by using an exonuclease; and
c) separating and purifying the linear closed DNA containing the gene of interest;
wherein the recombinant plasmid comprises prokaryotic telomerase target sequences and restriction endonuclease digestion sites, and the gene of interest is inserted between the prokaryotic telomerase target sequences.

10. The method according to claim 9, **characterized in that** the recombinant plasmid is the recombinant plasmid according to claim 8.

11. The method according to claim 9 or 10, **characterized in that** the exonuclease includes Exonuclease III and/or T5 Exonuclease.

12. The method according to claim 11, **characterized in that** the added amount of the exonuclease is 3000-6000 U/mg.

13. The method according to claim 12, **characterized in that** for the exonuclease, the digestion temperature is 34-40°C, and the digestion time is 2-12 hours; preferably, the digestion time is 3-6 hours.

14. The method according to claim 9 or 10, **characterized in that** after the digestion with the exonuclease, EDTA with a final concentration of 1-50 mM is added and the temperature is raised to 70-80°C for 5-30 minutes of treatment.

15. The method according to any one of claims 9-14, **characterized in that** after finishing the digestion with the prokaryotic telomerase and/or the digestion with the restriction endonuclease, the temperature is raised to 70-80°C for 5-30 minutes of treatment.

16. The method according to any one of claims 9-15, **characterized in that** the separation and purification method comprises one or any combination of gel filtration chromatography, anion exchange chromatography, concentration and liquid exchange, and sterilization filtration.

17. The method according to claim 16, **characterized in that** the separation and purification method comprises sequentially gel filtration chromatography and anion exchange chromatography.

18. The method according to claim 17, **characterized in that** a packing for the gel filtration chromatography is Bestarose 6FF, and an eluate from the first ultraviolet peak with an absorbance of ≥ 50 mAu is collected.

19. The method according to claim 18, **characterized in that** a packing for the anion exchange chromatography is Fractogel EMD DEAE.

20. The method according to claim 19, **characterized in that** loading conditions for the anion exchange chromatography comprise:
using a loading solution containing 30%-70% chromatographic solution B, with a loading capacity of 0.2-1.0 mg/mL and a loading flow rate of ≤ 120 cm/h, wherein
the chromatographic solution B comprises 0.5-1.5 M NaCl, 5-15 mM EDTA, and a buffer substance, pH 7.2-7.8.

21. The method according to claim 20, **characterized in that** elution conditions for the anion exchange chromatography comprise:
elution with 65%-100% chromatographic solution B at an elution flow rate of 30-120 cm/h, and collection of an eluate with an absorbance of ≥ 100 mAu.

22. A kit, **characterized by** comprising a prokaryotic telomerase and the plasmid according to any one of claims 1-7; or comprising a prokaryotic telomerase and the recombinant plasmid according to claim 8.

23. The kit according to claim 22, **characterized in that** the kit further comprises a restriction endonuclease and/or an exonuclease, wherein the restriction endonuclease is capable of recognizing the restriction endonuclease digestion site.

24. A linear closed DNA prepared by the method according to any one of claims 9-21.
